(19) Europäisches Patentamt
European Patent Office
Office européen des brevets

(11) EP 3 542 898 A1

(12) **EUROPÄISCHE PATENTANMELDUNG**

(43) Veröffentlichungstag:
**25.09.2019 Patentblatt 2019/39**

(21) Anmeldenummer: **19162261.2**

(22) Anmeldetag: **12.03.2019**

(51) Int Cl.:
***B01J 23/755** (2006.01)*
***B01J 21/12** (2006.01)*
***B01J 35/00** (2006.01)*
***B01J 37/02** (2006.01)*
***B01J 37/08** (2006.01)*
***C07C 2/10** (2006.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **14.03.2018 EP 18161764**

(71) Anmelder: **Evonik Degussa GmbH**
**45128 Essen (DE)**

(72) Erfinder:
- **Nadolny, Fabian**
  **59821 Arnsberg (DE)**
- **Peitz, Stephan**
  **45739 Oer-Erkenschwick (DE)**
- **Stochniol, Guido**
  **45721 Haltern am See (DE)**
- **Franke, Robert**
  **45772 Marl (DE)**
- **Quandt, Thomas**
  **45772 Marl (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

# (54) VERFAHREN ZUR HERSTELLUNG EINES OLIGOMERISIERUNGSKATALYSATORS

(57) Gegenstand der Erfindung ist ein Verfahren zur Herstellung eines Oligomerisierungskatalysators, welcher Nickeloxid und ein Silica-Alumina-Supportmaterial umfasst, wobei das Silica-Alumina-Supportmaterial röntgenamorph ist und in der Ammonium-Form vorliegt. Ein weiterer Gegenstand der vorliegenden Erfindung ist Verfahren zur Oligomerisierung von C3- bis C6-Olefinen unter Verwendung des erfindungsgemäß hergestellten Oligomerisierungskatalysators.

EP 3 542 898 A1

## Beschreibung

Die vorliegende Erfindung betrifft ein Verfahren zur Herstellung eines Oligomerisierungskatalysators, welcher Nickeloxid und ein Silica-Alumina-Supportmaterial umfasst, wobei das Silica-Alumina-Supportmaterial in der Ammonium-Form vorliegt. Ein weiterer Gegenstand der vorliegenden Erfindung ist Verfahren zur Oligomerisierung von C3- bis C6-Olefinen unter Verwendung des erfindungsgemäß hergestellten Oligomerisierungskatalysators.

Generell versteht man unter der Oligomerisierung die Reaktion von ungesättigten Kohlenwasserstoffen mit sich selbst, wobei entsprechend längerkettige Kohlenwasserstoffe, die so genannten Oligomere, entstehen. So lässt sich beispielsweise durch die Oligomerisierung von zwei Olefinen mit drei Kohlenstoffatomen ein Olefin mit sechs Kohlenstoffatomen (Hexen) aufbauen. Die Oligomerisierung von zwei Molekülen miteinander wird auch Dimerisierung genannt.

Die erhaltenen Oligomere sind Zwischenprodukte, die beispielsweise für die Herstellung von Aldehyden, Carbonsäuren und Alkoholen eingesetzt werden. Die Oligomerisierung von Olefinen wird großtechnisch entweder in homogener Phase an einem gelösten oder heterogen an einem festen Katalysator oder mit einem zweiphasigen Katalysatorsystem durchgeführt.

Bei den heterogen katalysierten Verfahren ist die Oligomerisierung an sauren Oligomerisierungskatalysatoren lange bekannt. Technisch werden z. B. Zeolithe oder Phosphorsäure auf einem Support eingesetzt. Hierbei werden Isomerengemische von verzweigten Olefinen erhalten. Für die nicht-saure, heterogen katalysierte Oligomerisierung von Olefinen, mit hoher Dimerenselektivität werden in der Technik häufig Nickelverbindungen auf Supportmaterialien eingesetzt. So wird in der WO 95/14647 A1 ein Nickelkatalysator mit einem Supportmaterial, das aus den Komponenten Titanoxid und/oder Zirkoniumoxid, Siliciumdioxid und gegebenenfalls Aluminiumoxid besteht, für die Olefinoligomerisierung beschrieben. An diesen Katalysatoren werden Gemische linearer Butene in einer Selektivität von unter 75 % zu C8-Olefinen oligomerisiert.

Für die Herstellung von Oligomerisierungskatalysatoren sind verschiedene Verfahren im Stand der Technik bekannt. Die WO 95/14647 A1 offenbart beispielsweise ein Verfahren zur Herstellung eines Oligomerisierungskatalysators, bei dem die einzelnen Komponenten aus einer Lösung ausgefällt werden. Die Zusammensetzung und Eigenschaften der eingesetzten Metallsalze sind dabei eher nebensächlich. Eine andere Möglichkeit die Oligomerisierungskatalysatoren herzustellen ist die das Mischen der entsprechenden Einzelkomponenten und die Granulation der Mischung mit gegebenenfalls nachträglichem Nickelauftrag mittels Imprägnierung.

Der vorliegenden Erfindung lag die Aufgabe zugrunde ein Verfahren bereitzustellen, mit dem ein Oligomerisierungskatalysator mit verbesserten Eigenschaften hergestellt werden kann, wobei kein negativer Effekt auf die Standzeit des Katalysators und die mechanischen Eigenschaften wie Festigkeit erfolgen darf.

Es hat sich überraschend gezeigt, dass die zugrundeliegende Aufgabe mit einem Verfahren zur Herstellung eines Oligomerisierungskatalysators gelöst werden konnte, bei dem das amorphe Silica-Alumina-Supportmaterial in der Ammonium-Form eingesetzt wird. Ein mit in der Ammonium-Form befindlichen Supportmaterial hergestellter Katalysator führt bei seinem Einsatz in der Oligomerisierung zu höheren Umsätze und zu höheren Selektivitäten zu linearen Produkten.

Das erfindungsgemäße Verfahren zur Herstellung eines Oligomerisierungskatalysators, umfassend Nickeloxid auf einem amorphen Silica-Alumina-Supportmaterial, umfasst die folgenden Schritte:

a) Mischen eines amorphen Silica-Alumina-Supportmaterials, welches 10 bis 20 Gew.-% $Al_2O_3$ und 80 bis 90 Gew.-% $SiO_2$ umfasst, eines Al-haltigen und Si-freien oder Al-freien und Si-haltigen Binders und optional mindestens eines Teils einer Nickelquelle, die eine wässrige, ammoniakfreie Nickellösung einer Nickelverbindung, eine wässrige, ammoniakfreie Nickel-Paste einer Nickelverbindung oder eine Kombination aus der vorgenannten Nickellösung und der vorgenannten Nickel-Paste ist, wobei die Nickelverbindung aus der Gruppe, bestehend aus Nickelnitrat ($Ni(NO_3)_2$), Nickelacetat ($Ni(ac)_2$), Nickelacetylacetonat ($Ni(acac)_2$), Nickelsulfat ($NiSO_4$), Nickelcitrat oder Nickelcarbonat ($NiCO_3$), ausgewählt wird, und Granulieren der so hergestellten Mischung;

b) Beaufschlagendes in Schritt a) hergestellten Granulats mit mindestens einem Teil einer wie für den Schritt a) definierten Nickelquelle, sofern die gesamte Nickelquelle nicht schon in Schritt a) mit dem amorphen Silica-Alumina-Supportmaterial und dem Binder vermischt worden ist; und

c) Kalzinieren des Granulats zur Herstellung des Oligomerisierungskatalysators;

dadurch gekennzeichnet dass das amorphe Silica-Alumina-Supportmaterial in Schritt a) in der Ammonium-Form vorliegt.

Mit dem Begriff Ammonium-Form im Sinne der vorliegenden Erfindung ist eine bestimmte Maskierung der Säurezentren im amorphen Silica-Alumina-Supportmaterial gemeint. Die Struktur des Silica-Alumina-Supportmaterials, insbesondere von Alumosilicaten, besteht üblicherweise aus $SiO_4$-Tetraedern und $AlO_4$-Tetraedern. An der Oberfläche

dieser Struktur fehlt bei Aluminiumatomen jedoch zur Umgebung hin der Sauerstoff von einem angrenzenden Tetraeder. Das Aluminiumatom weist deshalb formal nur eine trivalente Koordination mit einem freien Orbital auf (hier nicht gezeigt). Der Begriff Ammonium-Form meint den Zustand, bei dem ein Ammoniak-Molekül sich an das freie Orbital anlagert und sich formal mit dem Wasserstoff einer benachbarten Gruppe ein Ammonium-Molekül bildet (siehe die Struktur auf der linken Seite der nachfolgenden Abbildung). Im Gegensatz dazu meint der Begriff H-Form den Zustand, bei dem sich ein Sauerstoff einer benachbarten Gruppe an das freie Orbital anlagert, wodurch die O-H-Bindung destabilisiert wird und formal ein Proton ($H^+$) abgespalten werden kann (siehe die Struktur auf der rechten Seite der nachfolgenden Abbildung).

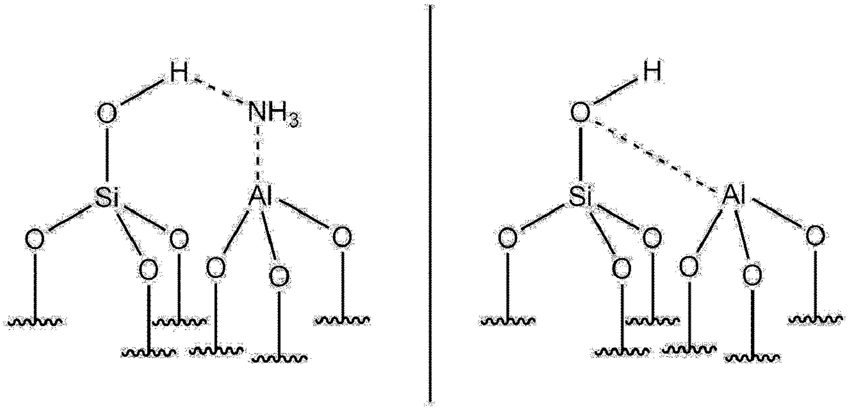

Das amorphe Silica-Alumina-Supportmaterial ist ein amorphes Alumosilicat. Mit "amorph" im Sinne der vorliegenden Erfindung ist die Eigenschaft eines Feststoffes gemeint, die daraus folgt, dass der Feststoff keine Kristallstruktur, d. h. keine Fernordnung, aufweist. Im Sinne der vorliegenden Erfindung ist aber nicht auszuschließen, dass das amorphe Silica-Alumina-Supportmaterial kleine kristalline Domänen aufweist. Das amorphe Silica-Alumina-Supportmaterial ist somit kein kristallines Material, beispielsweise kein zeolithisches Material.

Das Silica-Alumina-Supportmaterial ist ein amorphes Alumosilicat, welches 10 bis 20 Gew.-%, vorzugsweise 12 bis 17 Gew.-% $Al_2O_3$ und 80 bis 90 Gew.-%, vorzugsweise 83 bis 88 Gew.-% $SiO_2$ umfasst. Die Angabe bezieht sich auf die Zusammensetzung ohne eventuell sorbierte Verbindungen (z. B. Wasser oder Ammoniak), die beispielsweise unter dem Begriff Glühverlust in kommerziell erhältlichen Produkten angegeben werden. In einer bevorzugten Ausführungsform kann das als das Silica-Alumina-Supportmaterial eingesetzte amorphe Alumosilicat eine Korngröße (d50) im Bereich von 10 bis 80 μm, vorzugsweise 15 bis 75 μm aufweisen, gemessen mittels Laserbeugung, beispielsweise einem Mastersizer von Malvern. Darüber hinaus weist das als das Silica-Alumina-Supportmaterial eingesetzte amorphe Alumosilicat vorzugsweise eine spezifische Oberfläche (berechnet nach BET) von 250 bis 380 $m^2/g$, besonders bevorzugt von 280 bis 360 $m^2/g$ auf, gemessen mittels Stickstoff-Physisorption gemäß DIN-ISO 9277 (Stand: 2014-01). Der Anteil des Silica-Alumina-Supportmaterials an dem Gesamtansatz (Gesamtzusammensetzung inklusive aller ggf. verwendeten Lösemittel wie Wasser) in Schritt a) kann 20 bis 50 Gew.-%, vorzugsweise 25 bis 45 Gew.-% betragen, wenn die gesamte Nickelquelle bereits in Schritt a) zugegeben wird. Wird die Nickelquelle teilweise in Schritt b) zugegeben, sollte eine ausreichende Flüssigkeitsmenge durch Zugabe eines Lösemittels, vorzugsweise Wasser, zur Mischung in Schritt a) hinzugefügt werden, um eine Granulation zu ermöglichen.

Der in Schritt a) ebenfalls eingesetzte Binder kann in einer Ausführungsform ein Al-haltiger und Si-freier Binder (Si-frei bedeutet: <0,1 Gew.-% Si in der Gesamtzusammensetzung des Binders) sein. Der Al-haltige und Si-freie Binder ist vorzugsweise ein oxidisches Aluminiummaterial, vorzugsweise Aluminiumoxid, Aluminiumhydroxid, oder Aluminiumoxid-hydroxid, besonders bevorzugt Boehmit. Der Al-haltige und Si-freie Binder liegt weiterhin bevorzugt nicht in fester Form, sondern in gelöster Form, besonders bevorzugt als kolloidale Lösung, vor. Das Lösemittel, indem der Al-haltige und Si-freie Binder, vorzugsweise Aluminiumoxid, Aluminiumhydroxid, oder Aluminiumoxid-hydroxid, besonders bevorzugt Boehmit, in gelöster Form, vorzugsweise als kolloidale Lösung vorliegt, ist in einer bevorzugten Ausführungsform eine 1 Gew.-%ige Salpetersäurelösung. Der Al-haltige und Si-freie Binder ist in der Lösung, vorzugsweise der kolloidalen Lösung, in einer Menge im Bereich von 10 bis 25 Gew.-%, vorzugsweise 12 bis 20 Gew.-%, besonders bevozugt 14 bis 18 Gew.-%, vorhanden. Der Anteil des Al-haltigen und Si-freien Binders an dem Gesamtansatz (Gesamtzusammensetzung inklusive aller ggf. verwendeten Lösemittel wie Wasser) in Schritt a) kann 10 bis 30 Gew.-%, vorzugsweise 15 bis 25 Gew.-% betragen, wenn die gesamte Nickelquelle bereits in Schritt a) zugegeben wird. Sollte die Nickelquelle teilweise oder vollständig erst in Schritt b) zugegeben werden, beträgt der Anteil des Al-haltigen und Si-freien Binders an dem Gesamtansatz (Gesamtzusammensetzung inklusive aller ggf. verwendeten Lösemittel wie Wasser) in Schritt a) vorzugsweise 15 bis 50 Gew.-%, besonders bevorzugt 25 bis 40 Gew.-%.

Der in Schritt a) ebenfalls eingesetzte Binder kann in einer weiteren Ausführungsform ein Al-freier und Si-haltiger Binder (Al-frei bedeutet: <0,1 Gew.-% Al in der Gesamtzusammensetzung des Binders) sein. Der Al-freie und Si-haltige Binder ist vorzugsweise Siliciumdioxid. Der Al-freie und Si-haltige Binder liegt weiterhin bevorzugt nicht in fester Form, sondern in Form einer kolloidalen Dispersion, besonders bevorzugt einem Kieselsol, vor. Das Lösemittel, indem der Al-freie und Si-haltige Binder, vorzugsweise das Siliciumdioxid, dispergiert vorliegt, ist in einer bevorzugten Ausführungsform Wasser. Der Al-freie und Si-haltige Binder, vorzugsweise das Siliciumdioxid, ist in der Dispersion in einer Menge im Bereich von 7 bis 50 Gew.-%, vorzugsweise 12 bis 42 Gew.-%, besonders bevorzugt 20 bis 35 Gew.-% vorhanden. Die mittlere Partikelgröße des Al-freien und Si-haltigen Binders, vorzugsweise des Siliciumdioxids kann, insbesondere in der Dispersion, 5 bis 20, vorzugsweise 6 bis 10 nm betragen (kann mittels Lichtstreumethoden ermittelt werden). Die Viskosität der Dispersion mit dem Al-freien und Si-haltigen Binder, vorzugsweise dem Siliciumdioxid, kann im Bereich von 1 bis 50 mPas, vorzugsweise 5 bis 25 mPas liegen. Weiterhin bevorzugt kann die Dispersion mit dem Al-freien und Si-haltigen Binder, vorzugsweise dem Siliciumdioxid, einen pH-Wert im Bereich von 7 bis 12, vorzugsweise 8 bis 10 aufweisen. Die Dichte der Dispersion mit dem Al-freien und Si-haltigen Binder, vorzugsweise dem Siliciumdioxid, beträgt vorzugsweise 1 bis 1,3 g/cm$^3$, besonders bevorzugt1,1 bis 1,25 g/cm$^3$. Der Anteil des Al-freien und Si-haltigen Binders an dem Gesamtansatz (Gesamtzusammensetzung inklusive aller ggf. verwendeten Lösemittel wie Wasser) in Schritt a) und optional b) kann 0,5 bis 15 Gew.-%, vorzugsweise 1 bis 10 Gew.-% betragen.

Zur Mischung in Schritt a) kann zudem eine Alkalimetallverbindung, vorzugsweise eine Natriumverbindung, gegeben werden. Die Natriumverbindung ist vorzugsweise ein Natriumsalz, besonders bevorzugt Natriumcarbonat ($Na_2CO_3$). In einer besonders bevorzugten Ausführungsform wird das Natriumcarbonat als wässrige Lösung hinzugegeben. Die Menge an Natriumcarbonat (in nicht gelöster Form) an dem Gesamtansatz (Gesamtzusammensetzung aller ggf. verwendeten Lösemittel wie Wasser, also auch inklusive des zur Lösung des Natriumcarbonats ggf. verwendeten Wassers) in Schritt a) und optional b) des Herstellverfahrens kann zwischen 0,01 und 2,5 Gew.-%, vorzugsweise 0,05 und 2 Gew.-%, betragen.

Als Nickelquelle in Schritt a) oder b) kann prinzipiell jede lösliche Nickelverbindung eingesetzt werden. Darunter fallen Nickelnitrat ($Ni(NO_3)_2$), Nickelacetat ($Ni(ac)_2$), Nickelacetylacetonat ($Ni(acac)_2$), Nickelsulfat ($NiSO_4$), Nickelcitrat oder Nickelcarbonat ($NiCO_3$). Bevorzugt sind Nickelnitrat ($Ni(NO_3)_2$), Nickelsulfat ($NiSO_4$) und Nickelcarbonat ($NiCO_3$). Als Nickelquelle werden wässrige, ammoniakfreie Lösungen der vorgenannten Nickelverbindungen, wässrige, ammoniakfreie Pasten aus den vorgenannten Nickelverbindungen oder eine Kombination aus der vorgenannten Nickel-Lösung und der vorgenannten Nickel-Paste eingesetzt. Ammoniakfrei im Sinne der vorliegenden Erfindung bedeutet, dass die Nickelquelle im Wesentlichen frei von Ammoniak ist und höchstens Ammoniak als Verunreinigungen enthält. Der Nickelquelle als Lösung oder Paste wird kein Ammoniak zugesetzt. Die wässrige, ammoniakfreie Nickel-Paste enthält Wasser, wobei die Nickel-Paste gemäß der vorliegenden Erfindung weniger Wasser enthält als die Nickel-Lösung (wenn man von der gleichen Menge an Nickelverbindung ausgeht). Nickel-Paste ist prinzipiell ein angefeuchteter Feststoff aus einer Nickelverbindung, die unvollständig hydratisiert ist und bei der sich formal auch hydroxidische Nickelverbindung bilden, bei Nickelcarbonat beispielsweise $NiCO_3*Ni(OH)_2$, aber auch nicht-stöchiometrische Nickelcarbonat-Hydroxide. Die wässrige, ammoniakfreie Nickel-Paste enthält in einer bevorzugten Ausführungsform zwischen 30 und 50 Gew.-%, vorzugsweise 35 bis 45 Gew.-% Nickel bezogen auf das Gesamtgewicht der Paste. Die wässrige, ammoniakfreie Nickellösung kann Nickel in einer Menge im Bereich von 1 bis 20 Gew.-%, vorzugsweise 5 bis 15 Gew.-%, enthalten, jeweils bezogen auf das Gesamtgewicht der Lösung.

In einer bevorzugten Ausführungsform wird als Nickel-Lösung eine wässrige, ammoniakfreie $Ni(CO_3)$- oder $Ni(NO_3)_2$-Lösung, verwendet. Der Nickelgehalt in der Nickellösung kann von 1 bis 20 Gew.-%, vorzugsweise 5 bis 15 Gew.-% betragen. Als Nickel-Paste wird vorzugsweise eine Paste aus Nickelcarbonat und Wasser als Lösemittel verwendet, wobei das Nickel als Carbonat/Hydroxid vorliegt (allgemeine Summenformel $NiCO_3*Ni(OH)_2$, es können aber auch nicht-stöchiometrische Nickelcarbonat-Hydroxide gebildet werden). Die Paste kann einen Nickelgehalt im Bereich von 30 und 50 Gew.-%, vorzugsweise 35 bis 45 Gew.-% aufweisen.

In einer alternativen Ausführungsform werden bei der Herstellung des Oligomerisierungskatalysators (Schritt a) und/oder optional b)) sowohl eine Ni-Lösung als auch eine Nickelcarbonat-Paste verwendet. Das ist so zu verstehen, dass wenn die Zugabe der Nickelquelle ausschließlich im vorgenannten Schritt a) erfolgt, die Nickelquelle sowohl in Form einer Paste als auch in Form einer Lösung zugegeben werden kann. Andererseits ist das so zu verstehen, dass wenn die Zugabe der Nickelquelle nur teilweise oder gar nicht in Schritt a) sondern zumindest teilweise in Schritt b) erfolgt, die Nickelquelle in dem einen Schritt (a oder b) in Form einer Paste und im anderen Schritt (a oder b) in Form einer Lösung zugegeben werden kann oder in beiden Schritten (a oder b) sowohl in Form einer Paste als auch in Form einer Lösung zugegeben werden kann.

Die Gesamtmenge an Nickelquelle (wässrige, ammoniakfreie Paste und/oder wässrige, ammoniakfreie Lösung) an dem Gesamtansatz (Gesamtzusammensetzung aller ggf. verwendeten Lösemittel wie Wasser) in Schritt a) und optional b) des Herstellverfahrens kann zwischen 30 und 50 Gew.-%, vorzugsweise 35 und 45 Gew.-%, betragen.

Das erfindungsgemäße Verfahren zeichnet sich insbesondere dadurch aus, dass kein Titandioxid und kein Zirkoniumdioxid zugegeben werden, sondern der Oligomerisierungskatalysator ohne Hinzufügen von Titandioxid und

Zirkoniumdioxid hergestellt wird. Mögliche Vorkommen von Titandioxid und/oder Zirkoniumdioxid in der Gesamtzusammensetzung des Oligomerisierungskatalysators stammen aus Verunreinigungen bzw. Spurenvorkommen in den eingesetzten Komponenten.

Im Schritt a) werden die einzelnen Komponenten, d. h. das amorphe Silica-Alumina-Supportmaterial, der Binder und optional die Nickelquelle, miteinander in einem Mischbehälter unter Verwendung eines Wirblers vermischt und gleichzeitig oder anschließend granuliert. Dazu kann beispielsweise ein Intensivmischer verwendet werden. Die Vermischung und die Granulation können typischerweise bei Umgebungsdruck durchgeführt werden. Die Temperatur, bei der Vermischung und Granulation stattfinden können, liegt vorzugsweise im Bereich von 10 bis 60 °C. Die Dauer des Verfahrensschritts a), also der Vermischung und der Granulation, beträgt zwischen 5 Minuten und 1 Stunde, vorzugsweise zwischen 10 und 30 Minuten.

Im optionalen Schritt b) wird der restliche Teil der Nickelquelle, vorzugsweise in Form einer Ni-Lösung, zu dem in Schritt a) hergestellten Granulat gegeben und mit dem Granulat vermischt, um das Granulat mit Nickel zu beaufschlagen. Sollte zumindest ein Teil der Nickelquelle in Schritt b) zugegeben werden, kann das ggf. feuchte Granulat aus Schritt a) vor dem Beaufschlagen mit der Nickelquelle getrocknet werden. Die Trocknungstemperatur kann 80 bis 250 °C, vorzugsweise 100 bis 220 °C betragen.

Das aus Schritt a) und/oder Schritt b) resultierende Granulat kann noch mindestens einen Teil der verwendeten Lösemittel, also insbesondere Wasser, enthalten. Es kann sich also um ein feuchtes Granulat handeln. Bevor das gegebenenfalls noch feuchte Granulat der Kalzination in Schritt c) unterworfen wird, kann das feuchte Granulat gesiebt werden, vorzugsweise mit einem Sieb mit einer Maschenweite von 0,1 bis 1,5 mm. Der abgesiebte Teil des Granulats (Unterkorn) kann zurück zu Schritt a) der Granulation zurückgeführt werden.

Nach dem Vermischen und Granulieren in Schritt a), optional nach dem Beaufschlagen (Imprägnieren) des Granulats mit mindestens einem Teil einer Nickelquelle in Schritt b) und optional nach dem Sieben des feuchten Granulats kann das Granulat in Schritt c) zunächst getrocknet werden. Dazu können bekannte Geräte wie beispielsweise Bandtrockner oder ähnliches verwendet werden. Die Trocknungstemperatur kann im Bereich von 80 bis 250 °C, vorzugsweise im Bereich von 100 bis 220 °C liegen.

Bevor das optional getrocknete Granulat der Kalzination unterworfen wird, kann das getrocknete Granulat fraktioniert werden, um eine bestimmte Korngröße des Granulats einzustellen. Eine solche Fraktionierung kann beispielsweise durch die Verwendung mindestens eines Siebs mit einer definierten Maschenweite erreicht werden. In einer besonders bevorzugten Ausführungsform werden zwei Siebe verwendet, wobei das eine Sieb eine Maschenweite von 0,1 bis 1,5 mm und das andere Sieb eine Maschenweite von 2,5 bis 7 mm aufweist. Die übrigen Fraktionen (Ober- und Unterkorn) können ggf. nach vorheriger Mahlung zum Schritt a) zurückgeführt werden.

Nach der optionalen Trocknung und eventuellen Fraktionierung des Granulats erfolgt die Kalzination des Granulats. Dabei kann das Granulat in einem geeigneten Ofen, vorzugsweise im Stickstoffstrom, besonders bevorzugt Stickstoffgegenstrom, erhitzt werden. Dem Stickstoffstrom kann während der Kalzination Luft hinzugefügt werden, wobei die Menge der zugeführten Luft 100 bis 10000 Vol.-ppm (part per million volumetrisch), vorzugsweise 300 bis 7000 Vol.-ppm betragen kann. Die Kalzinationstemperatur kann 400 bis 800 °C, vorzugsweise 450 bis 700 °C, besonders bevorzugt 500 bis 600 °C betragen. Diese Temperatur kann über mehrere Stunden, vorzugsweise 5 bis 20 Stunde, besonders bevorzugt 8 bis 15 Stunden gehalten werden, bevor das Granulat abgekühlt wird. Beim Abkühlen kann in den Ofen Luft eingeleitet werden, die Menge der eingeleiteten Luft sollte aber kontrolliert werden. Die Menge der optional zugeführten Luft beträgt 100 bis 10000 Vol.-ppm, vorzugsweise 300 bis 7000 Vol.-ppm.

Danach kann das abgekühlte Granulat bzw. der fertige Oligomerisierungskatalysator, möglicherweise noch einmal fraktioniert werden, um eine bestimmte Korngröße des abgekühlten Granulats einzustellen. Eine solche Fraktionierung kann beispielsweise durch die Verwendung mindestens eines Siebs mit einer definierten Maschenweite erreicht werden. In einer besonders bevorzugten Ausführungsform werden zwei Siebe verwendet, wobei das eine Sieb eine Maschenweite von 0,1 bis 1,5 mm und das andere Sieb eine Maschenweite von 2,5 bis 7 mm aufweist. Die übrigen Fraktionen (Ober- und Unterkorn) können ggf. nach vorheriger Mahlung zum Schritt a) zurückgeführt werden.

Der so hergestellte Oligomerisierungskatalysator weist nach dem letzten Verfahrensschritt, der Kalzination bzw. einer nachgeschalteten Fraktionierung nach Abkühlung, eine finale Gesamtzusammensetzung von 15 bis 40 Gew.-%, vorzugsweise 15 bis 30 Gew.-% NiO, 10 bis 30 Gew.-% $Al_2O_3$, 55 bis 70 Gew.-% $SiO_2$ und 0,01 bis 2,5 Gew.-%, vorzugsweise 0,05 bis 2 Gew.-% eines Alkalimetalloxids auf. Die Angaben beziehen sich auf eine Gesamtzusammensetzung von 100 Gew.-%. In einer besonders bevorzugten Ausführungsform der vorliegenden Erfindung ist der Oligomerisierungskatalysator im Wesentlichen frei von Titandioxid und/oder Zirkoniumoxid, insbesondere enthält der Oligomerisierungskatalysator weniger als 0,5 Gew.-%, bevorzugt weniger als 0,1 Gew.-%, besonders bevorzugt weniger als 0,01 Gew.-% Titandioxid und/oder Zirkoniumdioxid in seiner Gesamtzusammensetzung.

In einer bevorzugten Ausführungsform weist der erfindungsgemäß hergestellte Oligomerisierungskatalysator eine Nickeldispersität auf, die ausgedrückt über die chemisorbierte Menge an CO mehr als 30 μmol/g, vorzugsweise mehr als 33 μmol/g, besonders bevorzugt mehr als 36 μmol/g beträgt.

Die Verteilung von Nickel auf der Oberfläche kann durch die Chemisorption von Kohlenmonoxid (CO) bestimmt

werden. Hierfür wird die Probe in einem ersten Schritt zunächst mit Wasserstoff reduziert. Danach wird CO über die Probe geleitet, bis kein CO mehr von der Probe aufgenommen wird. Anhand der daraus bestimmten Menge an chemisorbiertem CO lässt sich abschätzen, wie fein verteilt Nickel auf der Oberfläche vorliegt. Je feiner verteilt das Nickel vorliegt, desto mehr CO kann aufgenommen werden. Bei großen Nickelkristallen, also einer ungünstigeren Verteilung von Nickel, sind die Nickelatome im Inneren des Kristalls nicht für das CO zugänglich. Deshalb gilt, je feiner Nickel auf der Oberfläche verteilt ist, desto mehr CO kann bei gleicher Gesamtmenge an Nickel gebunden werden. Die Messung wurde wie folgt durchgeführt:
Von jeder Probe wurden 0,2 g in das Probengefäß des Messgeräts TPDRO 1100 von Thermo Scientific gefüllt. Damit elementares Nickel an der Oberfläche vorliegt, wurde die Probe mit Wasserstoff reduziert. Hierfür wurden 20 ml/min Argon mit einem Anteil an Wasserstoff von 5 % über die Probe geleitet und von Raumtemperatur auf 520 °C erhitzt und für 30 min gehalten. Das Gas wurde nach dem Verlassen des Probengefäßes über ein vor der Messung ausgeheiztes Molsieb zur Adsorption von Wasser geleitet.

Nach der Reduktion wurde die Probe unter inertem Gas auf Raumtemperatur abgekühlt und die CO-Chemisorption gestartet. Dafür wurden im Abstand von 10 min jeweils 366 μl CO-Gas über die Probe geleitet. Durch Chemisorption von CO am Nickel wird die Wärmeleitfähigkeit des Gases verändert, worüber die chemisorbierte Menge an CO quantifiziert werden kann. Es wurde so oft CO in Pulsen über die Probe geleitet, bis 5 aufeinanderfolgende Pulse keine Änderung der Wärmeleitfähigkeit zeigen, d. h. kein CO mehr aufgenommen wird.

Der erfindungsgemäß hergestellte Oligomerisierungskatalysator kann eine spezifische Oberfläche (berechnet nach BET) von 150 bis 400 $m^2$/g, vorzugsweise 190 bis 350 $m^2$/g, besonders bevorzugt von 220 bis 330 $m^2$/g aufweisen. Die BET-Oberfläche wird mittels Stickstoff-Physisorption gemäß DIN-ISO 9277 (Stand: 2014-01) gemessen.

In einer weiterhin bevorzugten Ausführungsform weist der mit dem erfindungsgemäßen Verfahren hergestellte Oligomerisierungskatalysator Mesoporen und Makroporen auf, hat also eine bimodale Porengrößenverteilung. Die Mesoporen des erfindungsgemäßen Oligomerisierungskatalysators weisen einen mittleren Porendurchmesser von 5 bis 15 nm, vorzugsweise von 7 bis 14 nm, besonders bevorzugt von 9 bis 13 nm auf. Die Makroporen des erfindungsgemäßen Oligomerisierungskatalysators weisen demgegenüber vorzugsweise einen mittleren Porendurchmesser von 1 bis 100 μm, besonders bevorzugt von 2 bis 50 μm auf. Das mittlere Porenvolumen des erfindungsgemäßen Oligomerisierungskatalysators, d. h. sowohl der Mesoporen als auch der Makroporen, kann 0,5 bis 1,5 $cm^3$/g, vorzugsweise 0,7 bis 1,3 $cm^3$/g betragen. Der mittlere Porendurchmesser und das mittlere Porenvolumen können mittels Quecksilber-Porosimetrie gemäß DIN 66133 (Stand: 1993-06) bestimmt werden.

Darüber hinaus kann der mittels des erfindungsgemäßen Verfahrens hergestellte Oligomerisierungskatalysator einen mittleren Partikeldurchmesser (d50) von 0,1 mm bis 7 mm, vorzugsweise 0,5 bis 6 mm, besonders bevorzugt von 1 mm bis 5 mm aufweisen. Der mittlere Partikeldurchmesser kann mittels bildgebender Verfahren ermittelt werden, insbesondere durch die in den Normen ISO 13322-1 (Stand: 2004-12-01) und ISO 13322-2 (Stand: 2006-11-01) ermittelt werden. Ein geeignetes Gerät zur Analyse des Partikeldurchmessers ist beispielsweise der Camsizer 2006 (Retsch Technology).

Der mit dem erfindungsgemäßen Verfahren hergestellte Katalysator kann insbesondere für die Oligomerisierung von C3- bis C6-Olefinen, vorzugsweise C3- bis C5-Olefinen, besonders bevorzugt C4-Olefinen oder darauf basierenden olefinhaltigen Einsatzgemischen verwendet werden. Die Olefine oder olefinhaltigen Einsatzgemische werden als Eduktstrom eingesetzt.

Gegenstand der vorliegenden Erfindung ist auch ein Verfahren zur Oligomerisierung von C3- bis C6-Olefinen, wobei olefinhaltiges Einsatzgemisch, welches die C3- bis C6-Olefine enthält, in mindestens einer Reaktionszone über einen Katalysator geleitet wird, wobei der erfindungsgemäß hergestellte Oligomerisierungskatalysator zur Katalyse der Oligomerisierungsreaktion eingesetzt wird.. Eine Reaktionszone umfasst erfindungsgemäß mindestens einen Reaktor und mindestens ein Destillationskolonne, in der die gebildeten Oligomere abgetrennt werden können. Das erfindungsgemäße Verfahren kann auch mit zwei oder mehr Reaktionszonen betrieben werden. Die Oligomerisierung findet vorzugsweise in flüssiger Phase statt.

Als Olefine für das erfindungsgemäße Verfahren werden C3- bis C6-Olefine, bevorzugt C3- bis C5-Olefine, besonders bevorzugt C4-Olefine oder darauf basierende olefinhaltige Einsatzgemische, die auch Anteile an analogen Alkanen enthalten können, eingesetzt. Geeignete Olefine sind unter anderem α-Olefine, n-Olefine und Cycloalkene. Bevorzugt sind die als Edukte eingesetzten Olefine n-Olefine. In einer besonders bevorzugten Ausführungsform handelt es sich bei dem Olefin um n-Buten. Der Begriff, darauf basierende olefinhaltige Einsatzgemische' ist erfindungsgemäß so zu verstehen, dass er jegliche Art von Gemischen betrifft, die die entsprechenden zu oligomerisierenden C3- bis C6-Olefine in einer Menge enthalten, die es ermöglicht, die Oligomerisierung durchzuführen. Bevorzugt enthalten die olefinhaltigen Einsatzgemische praktisch keine weiteren ungesättigten Verbindungen und mehrfach ungesättigte Verbindungen wie Diene oder Acetylenderivate. Vorzugsweise werden olefinhaltige Einsatzgemische eingesetzt, die weniger als 5 Gew.-%, insbesondere weniger als 2 Gew.-% an verzweigten Olefinen bezogen auf den Olefinanteil enthalten. Weiterhin bevorzugt werden olefinhaltige Einsatzgemische eingesetzt, die weniger als 2 Gew.-% verzweigte Olefine, insbesondere iso-Olefine, enthalten.

Propylen (C3) wird großtechnisch durch Spaltung von Naphtha hergestellt und ist eine Grundchemikalie, die leicht verfügbar ist. C5-Olefine sind in Leichtbenzinfraktionen aus Raffinerien oder Crackern enthalten. Technische Gemische, die lineare C4-Olefine enthalten, sind Leichtbenzinfraktionen aus Raffinerien, C4-Fraktionen aus FC- oder Steamcrackern, Gemische aus Fischer-Tropsch-Synthesen, Gemische aus Dehydrierung von Butanen und durch Metathese oder aus anderen technischen Prozessen entstandene Gemische. Beispielsweise können für das erfindungsgemäße Verfahren geeignete Gemische linearer Butene aus der C4-Fraktion eines Steamcrackers gewonnen werden. Dabei wird im ersten Schritt Butadien entfernt. Dies geschieht entweder durch Extraktion oder Extraktionsdestillation des Butadiens oder dessen Selektivhydrierung. In beiden Fällen wird ein praktisch butadienfreier C4-Schnitt erhalten, das Raffinat I. Im zweiten Schritt wird Isobuten aus dem $C_4$-Strom entfernt, z. B. durch Herstellung von Methyl-tert.-butylether (MTBE) durch Umsetzung mit Methanol. Andere Möglichkeiten sind die Umsetzung des Isobutens aus dem Raffinat I mit Wasser zu tert.-Butanol oder die sauer katalysierte Oligomerisierung des Isobutens zu Diisobuten. Der jetzt isobutenfreie C4-Schnitt, das Raffinat II, enthält wie gewünscht die linearen Butene und gegebenenfalls Butane. Optional kann noch das 1-Buten destillativ abgetrennt werden. Beide Fraktionen, die mit But-1-en oder die mit But-2-en, können im erfindungsgemäßen Verfahren eingesetzt werden.

In einer weiteren bevorzugten Ausführungsform werden C4-olefinhaltige Stoffströme dem Verfahren als olefinhaltige Einsatzgemische zugeführt. Geeignete olefinhaltige Einsatzgemische sind dafür unter anderem das Raffinat I (butadienfreier C4-Schnitt des Steamcrackers) sowie das Raffinat II (butadien- und isobutenfreier C4-Schnitt des Steamcrackers).

Eine weitere Möglichkeit, ein geeignetes Olefingemisch herzustellen, besteht darin, Raffinat I, Raffinat II oder ein ähnlich zusammengesetztes Kohlenwasserstoffgemisch in einer Reaktivkolonne zu hydroisomerisieren. Dabei kann u. a. ein Gemisch gewonnen werden, das aus 2-Butenen, geringen Anteilen 1-Buten und gegebenenfalls n-Butan sowie Isobutan und Isobuten besteht.

Die Oligomerisierung erfolgt in der Regel bei einer Temperatur im Bereich von 50 bis 200 °C, vorzugsweise 60 bis 180 °C, bevorzugt im Bereich von 60 bis 130°C, und bei einem Druck von 10 bis 70 bar, bevorzugt von 20 bis 55 bar. Sofern die Oligomerisierung in flüssiger Phase erfolgen soll, müssen die Parameter Druck und Temperatur hierfür so gewählt werden, dass der Eduktstrom (die eingesetzten Olefine oder Olefingemische) in flüssiger Phase vorliegt. Die gewichtbasierten Raumgeschwindigkeiten (Reaktandmasse pro Katalysatormasse pro Zeit; weight hourly space velocity (WHSV)) befinden sich im Bereich zwischen 1 g Reaktand pro g Katalysator und pro h (= 1 $h^{-1}$) und 190 $h^{-1}$, vorzugsweise zwischen 2 $h^{-1}$ und 35 $h^{-1}$, besonders bevorzugt zwischen 3 $h^{-1}$ und 25 $h^{-1}$.

In einer Ausführungsform beträgt der Grad der Dimerisierung (auch "prozentuale Selektivität bezogen auf die Dimerisierung" genannt) nach der Oligomerisierung bezogen auf das umgesetzte Edukt mindestens 60 %, weiter bevorzugt mindestens 75 %, besonders bevorzugt mindestens 80%.

Die Linearität eines Oligomerisierungsprodukts bzw. von den entstandenen Dimeren wird durch den ISO-Index beschrieben und stellt einen Wert für die mittlere Anzahl an Methylverzweigungen im Dimer dar. So tragen (für Buten als Edukt) z.B. n-Oktene mit 0, Methylheptene mit 1 und Dimethylhexene mit 2 zum ISO-Index einer C8-Fraktion bei. Je niedriger der ISO-Index ist, umso linearer sind die Moleküle in der jeweiligen Fraktion aufgebaut. Der ISO-Index errechnet sich nach folgender allgemeiner Formel:

$$\frac{(\text{einfach verzweigte Dimere (Gew.-\%)} + 2 \text{ x } \text{zweifach verzweigte Dimere (Gew.-\%)})}{100}$$

Demnach besitzt ein Dimerengemisch mit einem ISO-Index von 1,0 im Mittel genau eine Methylverzweigung pro Dimerenmolekül.

Der ISO-Index des Produkts aus dem erfindungsgemäßen Oligomerisierungsverfahren beträgt vorzugsweise 0,8 bis 1,2, besonders bevorzugt 0,8 bis 1,18.

Die nach dem erfindungsgemäßen Verfahren hergestellten Oligomere werden unter anderem zur Herstellung von Aldehyden, Alkoholen und Carbonsäuren genutzt. So ergibt beispielsweise das Dimerisat aus linearen Butenen durch Hydroformylierung ein Nonanalgemisch. Dieses liefert entweder durch Oxidation die entsprechenden Carbonsäuren oder durch Hydrierung ein C9-Alkoholgemisch. Das C9-Säuregemisch kann zur Herstellung von Schmiermitteln oder Sikkative verwendet werden. Das C9-Alkoholgemisch ist Vorstufe für die Herstellung von Weichmachern, insbesondere von Di-Nonyl-phthalaten oder DINCH.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen.

Nachfolgend wird die vorliegende Erfindung anhand von Beispielen näher erläutert. Alternative Ausführungsformen der vorliegenden Erfindung sind in analoger Weise erhältlich.

Beispiele:

Ein kommerziell erhältliches amorphes Alumosilicat wurde zunächst durch Versetzen mit einem Überschuss an ammoniumhaltiger Lösung und anschließendem Trocknen bei 100 bis 120 °C (nahezu) vollständig in die Ammonium-Form überführt, d.h. es wurde sichergestellt, dass alle Säurezentren durch Ammonium-Ionen maskiert sind.

Katalysator 1 (erfindungsgemäß):

In den Mischbehälter eines Intensivmischers wurde ein Binder (kolloidale Lösung mit Siliciumdioxid, $SiO_2$-Gehalt zwischen ca. 30 Gew.-%) und das amorphe Alumosilicat gegeben und verrührt. Die Rührung wurde gestoppt, sobald Granulate mit einem geeigneten Partikeldurchmesser (0,1 mm bis 7 mm) erhalten werden. Das so erhaltene Granulat wurde bei etwa 120 °C getrocknet und danach mithilfe von zwei Sieben gesiebt, um zu kleine oder zu große Partikel aus dem Granulat zu entfernen.

Das trockene Granulat wurde anschließend mit einer wässrigen $Ni(NO_3)_2$-Lösung (Ni-Gehalt zwischen 10 und 12,5 Gew.-%) imprägniert. Anschließend wird das Granulat in einem Ofen kalziniert. Für die Kalzination wird das Granulat auf eine Temperatur zwischen 500 bis 600 °C geheizt und diese Temperatur für etwa 10 bis 12 Stunden gehalten. Der mit Granulat gefüllte Ofen wird mit Stickstoffstoff durchströmt, wobei ein Verhältnis von Volumen an Granulat zu Volumen an Stickstoff pro Stunde (Normvolumen) von mindestens 1:1000 eingehalten wird. Während der Abkühlung des Granulats auf Raumtemperatur wurden ca. 6000 Vol.-ppm Luft in den Stickstoffstrom dosiert. Das abgekühlte Granulat entspricht dem fertigen Oligomerisierungskatalysator.

Katalysator 2 (nicht erfindungsgemäß):

Zur Herstellung von Katalysator 2 wurde das zunächst hergestellte Alumosilicat in Ammonium-Form bei 400 °C kalziniert. Dadurch wurde ein Teil der Ammonium-Kationen, die die Säurezentren maskiert haben, ausgetrieben. Das für die Herstellung des Katalysators eingesetzte Alumosilicat befindet sich also teilweise in der H-Form.

Die Herstellung von Katalysator 2 erfolgte dann gemäß Anspruch 1, mit dem Unterschied, dass das Alumosilicat - wie erwähnt - partiell in der H-Form befindet.

Katalysator 3 (nicht erfindungsgemäß):

Zur Herstellung von Katalysator 3 wurde das zunächst hergestellte Alumosilicat in Ammonium-Form bei 550 °C kalziniert. Dadurch wurde die gesamte Menge an Ammonium-Kationen, die die Säurezentren maskiert haben, ausgetrieben. Das für die Herstellung des Katalysators eingesetzte Alumosilicat befindet sich nahezu vollständig in der H-Form.

Die Herstellung von Katalysator 2 erfolgte dann gemäß Anspruch 1, mit dem Unterschied, dass das Alumosilicat - wie erwähnt - vollständig in der H-Form befindet.

Einsatz der Katalysatoren in der Oligomerisierung:

Es wurden jeweils ca. 12 g des Katalysators in ein Metallrohr mit einem Innendurchmesser vom 6 mm eingefüllt. Vor und hinter dem Katalysator wurden Glasperlen mit einem Durchmesser von 2 mm gegeben, die als Vorheiz- bzw. Abkühlphase dienen. Die Oligomerisierung wurde unter Verwendung von einem Buten/Butan-Gemisch mit 80 % n-Butenen bei 30 bar und einer Belastung von 7,5 g/h Buten pro Gram Katalysator durchgeführt, wobei die Reaktionstemperatur zwischen 80 °C und 100 °C variiert wurde. Die Produkte wurden gaschromatographisch auf den Umsatz an Butenen und die Linearität der Octene analysiert.

Die in Abhängigkeit von der Temperatur erzielten Umsätze und Selektivitäten für Katalysator 1 (erfindungsgemäß) und die nicht erfindungsgemäßen Katalysatoren 2 und 3, sowie die daraus resultierenden ISO-Indices sind in den Tabellen 1 bis 3 angegeben.

Tabelle 1: Umsätze und ISO-Indices bei der Oligomerisierung unter Verwendung von Katalysator 1

| Belastung (Zulauf C4-Olefine in g/h pro Masse Katalysator in g) als WSHV : 7,5 $h^{-1}$ | | | |
|---|---|---|---|
| | Temperatur | Umsatz | ISO-Index |
| | | bezogen auf C4-Olefine | |
| Katalysator 1 (erfindungsgemäß) | 80 °C | 37,4% | 1,18 |
| | 90 °C | 40,3% | 1,18 |
| | 100 °C | 45,6% | 1,16 |

Tabelle 2: Umsätze und ISO-Indices bei der Oligomerisierung unter Verwendung von Katalysator 2

| Belastung (Zulauf C4-Olefine in g/h pro Masse Katalysator in g) als WSHV : 7,5 $h^{-1}$ | | | |
|---|---|---|---|
| | Temperatur | Umsatz | ISO-Index |
| | | bezogen auf C4-Olefine | |
| Katalysator 2 (nicht erfindungsgemäß) | 80 °C | 27,1% | 1,20 |
| | 90 °C | 29,9% | 1,21 |
| | 100 °C | 33,9% | 1,23 |

Tabelle 3: Umsätze und ISO-Indices bei der Oligomerisierung unter Verwendung von Katalysator 3

| Belastung (Zulauf C4-Olefine in g/h pro Masse Katalysator in g) als WSHV : 7,5 $h^{-1}$ | | | |
|---|---|---|---|
| | Temperatur | Umsatz | ISO-Index |
| | | bezogen auf C4-Olefine | |
| Katalysator 3 (nicht erfindungsgemäß) | 80 °C | 20,7% | 1,30 |
| | 90 °C | 19,4% | 1,33 |
| | 100 °C | 30,0% | 1,37 |

Es zeigt sich überraschenderweise, dass wenn das amorphe Alumosilicat bei der Herstellung des Katalysators in der Ammonium-Form eingesetzt wird, deutlich bessere Umsätze und geringere ISO-Indices für das Produktgemisch erreicht werden könnten.

**Patentansprüche**

**1.** Verfahren zur Herstellung eines Oligomerisierungskatalysators, umfassend mindestens die Schritte:

a) Mischen eines amorphen Silica-Alumina-Supportmaterials, welches 10 bis 20 Gew.-% $Al_2O_3$ und 80 bis 90 Gew.-% $SiO_2$ umfasst, eines Al-haltigen und Si-freien oder Al-freien und Si-haltigen Binders und optional mindestens eines Teils einer Nickelquelle, die eine wässrige, ammoniakfreie Nickellösung einer Nickelverbindung, eine wässrige, ammoniakfreie Nickel-Paste einer Nickelverbindung oder eine Kombination aus der vorgenannten Nickellösung und der vorgenannten Nickel-Paste ist, wobei die Nickelverbindung aus der Gruppe, bestehend aus Nickelnitrat ($Ni(NO_3)_2$), Nickelacetat ($Ni(ac)_2$), Nickelacetylacetonat ($Ni(acac)_2$), Nickelsulfat ($NiSO_4$), Nickelcitrat oder Nickelcarbonat ($NiCO_3$), ausgewählt wird, und Granulieren der so hergestellten Mischung;
b) Beaufschlagendes in Schritt a) hergestellten Granulats mit mindestens einem Teil einer wie für den Schritt a) definierten Nickelquelle, sofern die gesamte Nickelquelle nicht schon in Schritt a) mit dem amorphen Silica-Alumina-Supportmaterial und dem Binder vermischt worden ist; und
c) Kalzinieren des Granulats zur Herstellung des Oligomerisierungskatalysators;

**dadurch gekennzeichnet, dass** das amorphe Silica-Alumina-Supportmaterial in Schritt a) in der Ammonium-Form

vorliegt.

2. Verfahren nach Anspruch 1, wobei in Schritt a) als Al-haltiger und Si-freier Binder ein oxidisches Aluminiummaterial oder als Al-freier und Si-haltiger Binder Siliciumdioxid eingesetzt wird.

3. Verfahren nach Anspruch 2, wobei als Al-haltiger und Si-freier Binder Aluminiumoxid, Aluminiumhydroxid oder Aluminiumoxid-hydroxid eingesetzt wird.

4. Verfahren nach einem der Ansprüche 1 bis 3, wobei die wässrige, ammoniakfreie Nickel-Paste zwischen 30 und 50 Gew.-% Nickel, bezogen auf das Gesamtgewicht der Nickel-Paste, enthält.

5. Verfahren nach Anspruch 4, wobei als wässrige, ammoniakfreie Nickel-Paste eine Paste aus Nickelcarbonat und Wasser als Lösemittel verwendet wird, wobei das Nickel als Carbonat/Hydroxid vorliegt.

6. Verfahren nach einem der Ansprüche 1 bis 5, wobei die wässrige, ammoniakfreie Nickellösung Nickel in einer Menge im Bereich von 1 bis 20 Gew.-%, bezogen auf das Gesamtgewicht der Lösung, enthält.

7. Verfahren nach Anspruch 6, wobei als wässrige, ammoniakfreie Nickellösung eine wässrige, ammoniakfreie $Ni(CO_3)$- oder $Ni(NO_3)_2$-Lösung verwendet wird.

8. Verfahren nach einem der Ansprüche 1 bis 7, wobei die Kalzination in Schritt c) bei einer Temperatur zwischen 400 °C und 800 °C durchgeführt wird.

9. Verfahren nach Anspruch 6, wobei die Kalzination in Schritt c) im Stickstoffstrom durchgeführt wird.

10. Verfahren nach einem der Ansprüche 1 bis 9, wobei der Oligomerisierungskatalysator eine finale Zusammensetzung von 15 bis 40 Gew.-%, vorzugsweise 15 bis 30 Gew.-% NiO, 10 bis 30 Gew.-% $Al_2O_3$, 55 bis 70 Gew.-% $SiO_2$ und optional 0,01 bis 2,5 Gew.-%, eines Alkalimetalloxids, aufweist.

11. Verfahren zur Oligomerisierung von C3- bis C6-Olefinen, wobei ein olefinhaltiges Einsatzgemisch, welches die C3- bis C6-Olefine enthält, in einer Reaktionszone über einen Katalysator geleitet wird, wobei der Katalysator ein gemäß dem Verfahren nach einem der Ansprüche 1 bis 10 hergestellter Oligomerisierungskatalysator zu Katalyse der Oligomerisierungsreaktion eingesetzt wird.

12. Verfahren zur Oligomerisierung nach Anspruch 11, wobei das olefinhaltige Einsatzgemisch weniger als 2 Gew.-% verzweigte Olefine enthält.

13. Verfahren zur Oligomerisierung Anspruch 11 oder 12, wobei die Oligomerisierung in flüssiger Phase stattfindet.

14. Verfahren zur Oligomerisierung nach einem der Ansprüche 11 bis 13, wobei die Oligomerisierung bei einem Druck von 10 bis 70 bar und einer Temperatur von 50 bis 200 °C durchgeführt wird, unter der Voraussetzung, dass, sofern die Oligomerisierung in flüssiger Phase durchgeführt wird, die Parameter Druck und Temperatur so gewählt werden, dass der Eduktstrom in flüssiger Phase vorliegt.

Europäisches Patentamt
European Patent Office
Office européen des brevets

# EUROPÄISCHER RECHERCHENBERICHT

Nummer der Anmeldung
EP 19 16 2261

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | HARMS S M ET AL: "Propene oligomerization over nickel-loaded silica-alumina", FUEL PROCESSING TECHNOLOGY, ELSEVIER BV, NL, Bd. 21, Nr. 3, 1. März 1989 (1989-03-01), Seiten 231-243, XP023646372, ISSN: 0378-3820, DOI: 10.1016/0378-3820(89)90052-0 [gefunden am 1989-03-01] * Seiten 233-235; Tabelle 2 * * Seite 241, Absatz 2; Abbildung 1; Tabelle 3 * ----- | 1-14 | INV. B01J23/755 B01J21/12 B01J35/00 B01J37/02 B01J37/08 C07C2/10 |
| Y | WO 2016/067033 A1 (BATTELLE MEMORIAL INSTITUTE [US]; BROWN FRASER [GB]) 6. Mai 2016 (2016-05-06) * Absatz [0102]; Anspruch 38; Beispiel 7 * ----- | 1-14 | |
| Y | FR 3 054 455 A1 (IFP ENERGIES NOW [FR]) 2. Februar 2018 (2018-02-02) * Seite 3, Zeilen 26-29 * * Seite 11, Zeile 16 - Seite 12, Zeile 10 * * Beispiele 1B,2B,4 * ----- | 1-14 | RECHERCHIERTE SACHGEBIETE (IPC) B01J C07C |
| Y | WO 2010/117539 A2 (UOP LLC [US]; NICHOLAS CHRISTOPHER P [US]; BHATTACHARYYA ALAKANANDA [U) 14. Oktober 2010 (2010-10-14) * Absätze [0038], [0040]; Beispiele 2,3,7,8 * ----- | 1-14 | |
| Y | DE 10 2009 027408 A1 (EVONIK OXENO GMBH [DE]) 5. Januar 2011 (2011-01-05) * Absätze [0003], [0037], [0044], [0045]; Beispiele 1,2 * ----- -/-- | 1-14 | |

Der vorliegende Recherchenbericht wurde für alle Patentansprüche erstellt

| Recherchenort | Abschlußdatum der Recherche | Prüfer |
|---|---|---|
| München | 7. August 2019 | Goebel, Matthias |

1

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 19 16 2261

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | WO 2014/123243 A1 (JX NIPPON OIL & ENERGY CORP [JP]) 14. August 2014 (2014-08-14)<br>* Zusammenfassung *<br>* Seite 3; Beispiele 1-3 *<br>----- | 1-14 | |
| Y | US 3 557 242 A (SAMPSON ROY JOHN ET AL) 19. Januar 1971 (1971-01-19)<br>* Tabelle Spalten 7/8, Runs 15-17, note 10;<br>Ansprüche; Beispiel 5 *<br>----- | 1-14 | |
| Y | US 2 581 228 A (BAILEY GRANT C ET AL) 1. Januar 1952 (1952-01-01)<br>* Spalte 9, Zeilen 22-24; Beispiele 3,6 *<br>----- | 1-14 | |
| A | EMIEL J.M. HENSEN ET AL: "Acidity Characterization of Amorphous Silica-Alumina",<br>JOURNAL OF PHYSICAL CHEMISTRY C,<br>Bd. 116, Nr. 40,<br>18. September 2012 (2012-09-18), Seiten 21416-21429, XP055511134,<br>ISSN: 1932-7447, DOI: 10.1021/jp309182f<br>* das ganze Dokument *<br>----- | 1-14 | RECHERCHIERTE SACHGEBIETE (IPC) |

Der vorliegende Recherchenbericht wurde für alle Patentansprüche erstellt

1

| Recherchenort | Abschlußdatum der Recherche | Prüfer |
|---|---|---|
| München | 7. August 2019 | Goebel, Matthias |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 19 16 2261

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-08-2019

| Im Recherchenbericht angeführtes Patentdokument | Datum der Veröffentlichung | Mitglied(er) der Patentfamilie | Datum der Veröffentlichung |
|---|---|---|---|
| WO 2016067033 A1 | 06-05-2016 | AU 2015340298 A1 | 27-04-2017 |
| | | BR 112017007326 A2 | 27-03-2018 |
| | | CA 2963749 A1 | 06-05-2016 |
| | | CN 107148461 A | 08-09-2017 |
| | | EP 3212737 A1 | 06-09-2017 |
| | | JP 2017537994 A | 21-12-2017 |
| | | US 2016194257 A1 | 07-07-2016 |
| | | US 2017218283 A1 | 03-08-2017 |
| | | WO 2016067033 A1 | 06-05-2016 |
| FR 3054455 A1 | 02-02-2018 | KEINE | |
| WO 2010117539 A2 | 14-10-2010 | BR PI1013710 A2 | 05-04-2016 |
| | | CN 102448913 A | 09-05-2012 |
| | | CN 104276911 A | 14-01-2015 |
| | | EP 2414310 A2 | 08-02-2012 |
| | | JP 5553889 B2 | 16-07-2014 |
| | | JP 2012522109 A | 20-09-2012 |
| | | KR 20120001795 A | 04-01-2012 |
| | | TW 201040251 A | 16-11-2010 |
| | | WO 2010117539 A2 | 14-10-2010 |
| DE 102009027408 A1 | 05-01-2011 | DE 102009027408 A1 | 05-01-2011 |
| | | WO 2011000697 A1 | 06-01-2011 |
| WO 2014123243 A1 | 14-08-2014 | JP 2014151253 A | 25-08-2014 |
| | | WO 2014123243 A1 | 14-08-2014 |
| US 3557242 A | 19-01-1971 | BE 729350 A | 04-09-1969 |
| | | DE 1911030 A1 | 25-09-1969 |
| | | FR 2003215 A1 | 07-11-1969 |
| | | GB 1195307 A | 17-06-1970 |
| | | NL 6903344 A | 08-09-1969 |
| | | US 3557242 A | 19-01-1971 |
| US 2581228 A | 01-01-1952 | GB 619231 A | 07-03-1949 |
| | | US 2581228 A | 01-01-1952 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9514647 A1